# EUROPEAN PATENT SPECIFICATION

(11) **EP 2 282 203 B1**
(45) Date of publication and mention of the grant of the patent: **14.03.2012**
(21) Application number: 10171772.6
(22) Date of filing: 03.08.2010
(51) Int. Cl.: G01N 31/10, G01N 33/38

(54) **Method of measuring the photocatalytic activity of cementitious materials**
Verfahren zur Messung der fotokatalytischen Aktivität von Zementmaterialien
Procédé de mesure d'activité photocatalytique de matériaux cimentaires

(30) Priority: 04.08.2009 IT MI20091415
(43) Date of publication of application: 09.02.2011
(73) Proprietor: ITALCEMENTI S.p.A., 24121 Bergamo (IT)
(72) Inventor: Borzetti, Fabio, I-00154 Roma (IT); Guerrini, Gian Luca, I-20063 Cernusco sul Naviglio (IT); Cassar, Luigi, I-20097 San Donato Milanese (IT); Campanella, Luigi, I-00141 Roma (IT); Visco, Giovanni, I-00100 Roma (IT)
(74) Representative: Gerli, Paolo

(56) References cited:
- US-A1- 2006 123 885
- STRINI A ET AL: "Measurement of benzene, toluene, ethylbenzene and o-xylene gas phase photodegradation by titanium dioxide dispersed in cementitious materials using a mixed flow reactor" APPLIED CATALYSIS B: ENVIRONMENTAL, ELSEVIER, vol. 61, no. 1-2, 27 October 2005 (2005-10-27), pages 90-97, XP025331861 ISSN: 0926-3373 [retrieved on 2005-10-27]
- RUOT B ET AL: "TiO2-containing cement pastes and mortars: Measurements of the photocatalytic efficiency using a rhodamine B-based colourimetric test" SOLAR ENERGY, PERGAMON PRESS. OXFORD, GB, vol. 83, no. 10, 28 July 2009 (2009-07-28) , pages 1794-1801, XP026584938 ISSN: 0038-092X [retrieved on 2009-07-28]
- HUNGER M ET AL: "Photocatalysis applied to concrete products part 1: Principles and test procedure" ZKG INTERNATIONAL, BAUVERLAG BV., G]TERSLOH, DE, vol. 61, no. 8, 1 August 2008 (2008-08-01) , pages 77-85, XP001517747 ISSN: 0949-0205

## Description

### Field of application

The present invention relates to the field of photocatalysis and in particular of building materials comprising photocatalysing substances.

### Prior art

Photocatalysts are generally semiconductors which have an energy difference between the conduction band (CB) and the valence band (VB), which can be overcome by means of UV irradiation. The value of the energy gap (Eg) between the two bands depends on various factors, primarily the nature of the semiconductor.

When the surface of the photocatalyst is illuminated, the photons with an energy greater than that of the energy gap (Eg) may be absorbed by the electrons found in the valence band of the semiconductor. The photoexcited electrons which have sufficient energy can be promoted by the valence band to the conduction band with higher energy. With this mechanism an electron-hole pair (e⁻/h⁺) is created which can be exploited chemically to activate for example redox reactions, also through the formation of oxygen radicals (oxide and superoxide).

Titanium dioxide (TiO₂) is a semiconductor, with Eg equal to approximately 3 eV. The TiO₂ is activated by a radiation of incident light with wavelength equal to approximately 400 nm, corresponding to the near UV. More particularly, the anatase form of the TiO₂ has an energy difference between CB and VB of 3.23 eV corresponding to a wavelength of 384 nm; the rutile form of the TiO₂ has an energy difference between the two bands of 3.02 eV corresponding to a wavelength of 411 nm.

The abovementioned properties of TiO₂ are also manifested when it is present, at the surface or mixed in bulk, in cementitious products: these products therefore behave like actual photocatalysts.

Cements with photocatalytic activity and containing TiO₂ have been known for some time and have numerous applications, for example in road pavings, in the walls of buildings, in tunnel vaults, etc. (see for example WO98/05601 and WO2004/074202). Thanks to the photocatalytic activity they are able, in the presence of environmental humidity and light, to decompose through contact a wide range of atmospheric pollutants, including, for example, nitrogen oxides.

From the point of view of quality control in production, and of the monitoring of the performances of products in place, it is interesting to be able to measure the extent of the photocatalytic activity of a cementitious material containing TiO₂ or other photocatalysts. The activity may in fact vary, at times to an unpredictable extent, on the basis of various factors including the concentration, the level of dispersion, the specific surface, the level of cleanliness of the surface, etc.

Some methods for measuring the activity of photocatalysts have been described. They are based in general on the use of appropriate indicators which are decomposed by the photocatalyst, in proportion to its activity. Among the indicators it is possible to use coloured organic substances: the decomposition is reflected in a modification of the spectrum of absorption of the indicator, which can be related to the extent of the photocatalytic activity. Other methods provide for a direct quantification of the mass of residual indicator or of its decomposition products. Some authors propose the use of alkaline halides which, following a reduction reaction catalysed by the photocatalyst, produce a quantity of hydroxide ions and therefore a variation of pH proportional to the photocatalytic activity. Systems have also been described which comprise an appropriate sensor to be placed at a short distance from the surface of the photocatalyst; the sensor is able to detect free radicals generated by the photo-oxidation of an indicator, whose quantity is proportional to the activity of the photocatalyst. Strini et al. (Applied Catalysis B, Vol.61 (2005), 90-97) describe the measurement of photocatalytic activity by analysing the composition of a gas that has passed a cementitious material containing photocatalysts.

All the aforementioned methods have limitations from the point of view of practicality and are often difficult to carry out on articles of manufacture already in place. More particularly the reactions which involve the use of indicators often function only in solution or require the formation of appropriate films, which makes their use impossible on structures already formed, for example cementitious articles of manufacture. Even when it may be possible, the addition of indicators to the article of manufacture is an inconvenient method and can cause damage to the surface of the same. The sensors for radicals require a sophisticated and costly technology and the recording of these species in a complete and reproducible manner is difficult. Moreover all the methods mentioned above give an indication of relative photocatalytic power, i.e. determined by the capacity of decomposition of a certain chemical species or on the detection of specific decomposition elements: they do not give an absolute indication of the photocatalytic properties, i.e. of the intensity with which the basic mechanism of photoexcitation acts, at the basis of photocatalysis.

The need is therefore felt for methods of measuring photocatalytic activity which are sensitive, reliable and cost-effective. These methods should be applicable with ease to the articles of manufacture to be tested, both during production and during their service life. A method is also required which allows an absolute valuation of the photocatalytic activity. These needs are particularly felt in the field of photocatalytic cements and, more specifically, in those containing titanium dioxide, in that photocatalysts are widely applied in this sector. There is finally the need for methods that are particularly suitable (in terms of stability and sensitivity) for cementitious products, which are often characterised by a relatively low rate of TiO₂ in the dispersed state in the bulk of the product or layered on its surface.

### Summary of the invention

The object of the present invention is a method of measuring the photocatalytic activity of cementitious materials containing photocatalysts, comprising the measuring of the electromotive force generated between two electrodes placed in contact respectively with an illuminated area and a darkened area of said material. The method is accurate, easy to apply and able to detect and distinguish levels of photocatalytic activity as commonly generated by cements containing photocatalysts.

### Description of the drawings

Figure 1: measuring of the EMF vs. time for the specimen of cement 1 containing photocatalyst (5% TiO₂) subjected to UV/dark cycle.
Figure 2: measuring of the EMF vs. time for the specimen of cement 1B not containing photocatalyst subjected to UV/dark cycle.
Figure 3: measuring of the EMF vs. time of day for specimen containing photocatalyst (5% TiO₂) subjected to the environmental light cycle.

### Detailed description

The present invention is based on the finding that, between two electrodes placed respectively in an illuminated area and a darkened area of a cementitious material containing a photocatalyst, an electromotive force (EMF) is established of intensity proportional to the movement of the electrons of the photocatalyst between the valence band and the conduction one. This flow, in that independent of the reaction of decomposition of specific materials, gives a non-relative indication of the photocatalytic properties of the material in question.

The method in question is suitable for measuring the activity of any material containing a photocatalyst. The term "photocatalyst" used in this application is not to be understood to be limited to single photocatalysts but includes also the combinations of two or more compounds with photocatalytic activity. The method is preferably applied to materials comprising the photocatalyst titanium dioxide (TiO₂); the TiO₂, or at least part thereof, is preferably present in crystalline anatase form.

The method according to the invention allows the detection of the level of activity of the photocatalyst, whether dispersed in bulk in the material, layered on the surface or present in any other form. The method has proved particularly accurate and sensitive, more particularly in the measurement of activity of materials containing TiO₂ in weight concentrations comprised between 0.5% and 10%, as is normally the case for photocatalytic cements.

The cementitious material whereto the present method is applicable can be without limitation a material comprising at least one hydraulic binder and at least one photocatalyst; it is preferably a mortar, a concrete, a plaster, a cementitious coating or a cementitious paint. The cementitious material is tested in the consolidated and dry state. It is typically in the form of an article of manufacture, e.g. a paving or an element thereof (e.g. tile), a wall or an element thereof (e.g. brick), a vault of a tunnel or an element thereof, a sample, a specimen taken from an article of manufacture in place, etc. In a particular embodiment the article of manufacture is large in size (architectural element).

The activity of the photocatalyst can be measured by connecting with each other two suitable electrodes placed respectively in an illuminated area and in a darkened area of the material to be tested. The EMF generated can be measured by means of a multimeter or other similar system placed along the path of connection between the two electrodes. In the present method there is no need to apply electrical energy to the specimen tested; the consumption of energy is therefore limited to the actuation of the lamp and of the EMF meter.

The electrodes are preferably made up of an electrically conductive material (graphite, aluminium, copper, etc.). They can be stably integrated in the material to be tested or can be placed in extemporaneous contact with it at the time of measurement: this contact can be formed by means of the use of electrodes with collet or made in any other way so as to ensure a stable and electrically conductive contact between the electrode and the surface or the bulk of the specimen to be tested. The electrodes can be positioned at the surface on the same face or on opposite faces of the specimen in question. The distance between the electrodes may vary widely; by way of a non-limiting example, useful values of distance for obtaining values of EMF which can be recorded with standard instruments are those comprised within the range between 1 and 10 mm, e.g. 6 mm.

A fundamental part of the method is the differentiation of conditions of lighting between the two areas containing the electrodes: in fact the photocatalysis takes place, to a significant extent, only in the presence of light. Therefore an adequate differentiation of light intensity between the two areas is necessary for supplying an EMF which can be detected and for allowing measurement. The source of illumination comprises the wavelength corresponding to the energy gap (Eg) of the catalyst in question. It can also be the environmental one (solar), however it is preferable to illuminate the specimen with a constant and controlled energy source: for materials containing TiO₂, it is preferably comprised between 350 nm and 400 nm; more preferably, in the case of materials containing TiO₂ in anatase form, it is equal to 384 nm. The darkening of the other portion of specimen can be carried out by any method. The darkening is preferably complete, however the method can give a significant indication of the photocatalytic activity also in conditions of non-total darkening, on condition that a significant differential of light intensity is maintained at the wavelength concerned between the two surfaces involved.

The illumination/darkening in controlled conditions is easily carried out in lab conditions by placing the area of the specimen to be illuminated under a lamp with the appropriate wavelength and the specimen to be darkened in a closed and non-illuminated room. Should the specimen to be analysed be already in place, it will be possible for example to take a portion thereof and subject it to investigation in the laboratory in the ways described above. Alternatively it will be possible to perform the measurements directly in field, i.e. on the article of manufacture in place, for example by setting up on the two areas concerned suitable domes not transparent to light, or other functionally similar shapes: the first thereof is provided with illumination at the wavelength concerned and the other is without illumination. The domes are integrated/integrable with the relative electrode and connected/connectable to each other by means of conductive material connected to a multimeter or other system for the measuring of the EMF. For the area to be darkened, the dome solution can be replaced by the apposition of any other solid body not transparent to the light.

The method of measurement comprises, preferably, an antecedent phase of conditioning wherein both the areas of the article of manufacture involved in the measurement are maintained in identical conditions of darkness, generally for a period of time comprised between 2 and 24 hours. Subsequently one of the two areas is illuminated, maintaining the other one in unvaried conditions of darkness. The illumination is protracted for a set period of time, e.g. approximately 60 minutes, up to stabilisation of the signal, then the final reading of the EMF is performed.

The present method can be used in various ways. For example it can be exploited to follow on a continuous basis the trend of the photocatalytic activity during a set interval of time (a day, a week, a month, etc.). Otherwise it is possible to valuate the variations of efficiency of the article of manufacture by extemporaneously measuring the activity at a preset time of the day (or of the week, month, etc.) and repeating this measurement for all the days (or weeks, months, etc.) until the timespan concerned is covered. In all these cases it may be sufficient to use as reference the activity of the specimen as resulting from the first measurement and monitor over time if and by how much the activity is reduced or in any case modified.

More accurate quantitative valuations can be obtained by performing measurements in parallel on articles of manufacture identical to the one tested yet without photocatalyst; this method allows to detract, from the signal detected, the possible background noise generated by electrical fields present within the material tested, obtaining in this way a more accurate indication of the sole contribution due to the photocatalyst.

For new materials (understanding "new" to be newly produced materials or in any case appropriately conserved and not subjected to wear and/or pollution), the measurement of the photocatalytic activity is useful for the purposes of quality control of the finished product. The method can also be used as a means of control in the research and development of new photocatalytic products, for which certain target levels of photocatalytic activity are to be obtained.

In the case wherein the titre of activity of the photocatalyst as such has been ascertained previously, the method according to the invention can be used to measure the concentration of photocatalyst present in the specimen analysed: in this case it will be possible to trace the concentration of the specimen by means of appropriate calibration curves. Naturally this measurement is meaningful on new or in any case appropriately conserved specimens, for which variations in photocatalytic activity due to wear can be excluded. For the used materials ("used" understood to mean construction materials that are not recent and/or conserved in inadequate conditions and/or subjected to wear and/or pollution, such as the case of the articles of manufacture put in place for a certain time), the measurement gives an indication of the possible decrease or other change in the photocatalytic activity, and therefore of the possible need to restore the photocatalytic element involved. The restoring may take place through simple cleaning of the surface, treatment of the surface with appropriate fluid materials (paints, grouts, mortars, etc.) containing the photocatalyst, or total replacement of the element itself with a new one. The measurement of the activity of new articles of manufacture or those already put in place is of primary interest: it in fact allows detecting a possible loss of efficiency of the article of manufacture and therefore a potential environmental accumulation of non-decomposed pollutants.

The invention is now illustrated by means of the following examples which do not have a function of limitation.

### Experimental part

### PREPARATION OF THE SPECIMENS

A specimen (no. 1) of circular shape (diameter 9 cm, thickness 6 mm) was prepared using the TX Aria (Italcementi) cement containing 5% TiO₂. A specimen of identical shape (1B) was prepared as reference using Italbianco (Italcementi) cement, free of TiO₂. Both specimens were made by basing on water/cement mixtures equal to 0.5, cured for 28 days. Before use all samples were sanded with fine sandpaper.

### Measurement procedure

Two electrodes of conductive material (graphite) were placed in contact with two different areas of the specimen to be tested. An optical fibre, positioned 15 cm from the surface of the cement, is set up to transmit from a UV lamp the light necessary for the activation of the photocatalytic cement.

Before the analysis the specimen is kept completely in the dark and after a prefixed time the predetermined area of the surface of the cement is irradiated with UV light. At the time of irradiation an electrical voltage is created between the illuminated area and the one remaining in the dark. The value of EMF over time, measured by means of a digital laboratory multimeter and recorded by means of a data logger with the computer, is prolonged until stabilisation of the signal. At this point the reference datum is taken and the irradiation is interrupted. This procedure was repeated for both the specimens 1 and 1B.

### Results

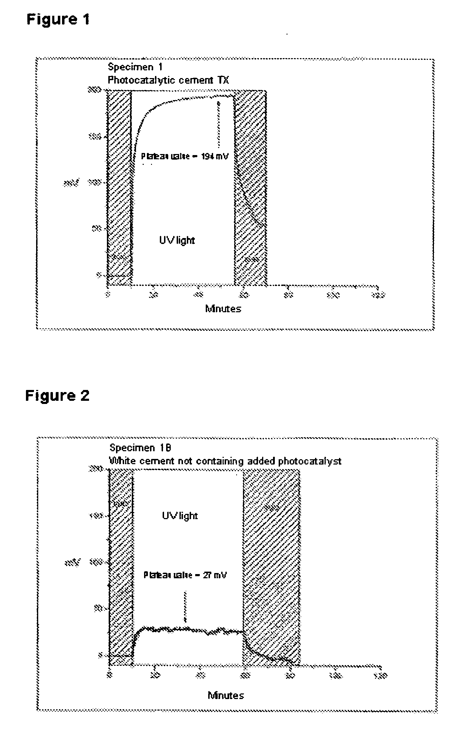

Figures 1 and 2 show two graphs obtained by applying the methodology previously described to the specimens 1 and 1B. In the case of specimen 1 the irradiation causes a rise in the electromotive force which in a short time stabilises at 194 mV. In the case of the non-photocatalytic specimen instead the EMF stabilises at the basal value of 27 mV. Note that the specimen containing 5% photocatalyst generates a distinctly higher EMF.

In order to ascertain the sensitivity/repeatability of the method the specimens were subjected 5 times to the same procedure of irradiation. The results obtained, all contained within a maximum range of ± 3% in relation to the initial value, confirmed the sensitivity and the reproducibility of the method in respect of the specimens tested.

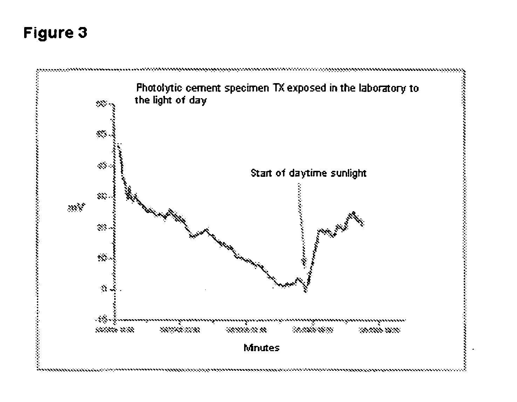

Figure 3 shows the use of the method in the continuous monitoring of the photocatalytic activity of an article of manufacture put in place, exposed to the environmental cycle of light/dark. The two measuring electrodes were applied respectively to an area exposed to environmental illumination and to another area appropriately covered with light insulating material. The trend of the photocatalytic activity during the period examined (6.30 p.m. - 10.30 a.m.) corresponds to the one expected as a function of the light/dark cycle whereto the specimen was subjected: i.e. the activity decreases during the sunset hours, reaching a minimum during the night time, and then recovering noticeably at the start of the morning. This item of data shows the capacity of the present method for providing a signal of EMF proportional to the intensity of photocatalytic activity exerted by the material being examined.

## Claims

1. Method of measuring the photocatalytic activity of a cementitious material containing photocatalysts, comprising measuring the electromotive force generated between two electrodes placed in contact respectively with an illuminated and a darkened area of said material.

2. Method according to claim 1, wherein the photocatalyst comprises titanium dioxide, present at least partly in anatase form.

3. Method according to claims 1-2, wherein the photocatalyst is dispersed in bulk in the material or is layered on its surface.

4. Method according to claims 1-3, wherein the photocatalyst is present, in bulk in the material or in a phase layered on its surface, in a weight concentration comprised between 0.5% and 10%.

5. Method according to claims 1-4, wherein the cementitious material is a mortar, a concrete, a plaster, a cementitious coating, or a cementitious paint.

6. Method according to claims 1-5, wherein the cementitious material is an article of manufacture, e.g. a paving or an element thereof, a wall or an element thereof, a tunnel vault or an element thereof, a sample, or a specimen taken from an article of manufacture in place.

7. Method according to claims 1-6, wherein the electromotive force is measured by means of a multimeter connected to the two electrodes.

8. Method according to claims 1-7, wherein the photocatalyst is titanium dioxide and the illuminated area is irradiated with a wavelength comprised between 350 nm and 400 nm.

9. Method according to claims 1-8, performed in continuous during a determined period of time.

10. Method according to claims 1-8, performed via a number of repeated observations.

11. Method according to claims 1-10, wherein the electromotive force of a photocatalyst-free sample equivalent to the tested one is deducted from the measured electromotive force.

12. Method according to claims 1-11, wherein the measured level of photocatalytic activity is used to monitor the quality of newly produced cementitious materials.

13. Method according to claims 1-11, wherein the measured level of photocatalytic activity is used to monitor the quality of cementitious materials already in place and subjected to wear.

14. Method according to claims 1-11, wherein the measured level of photocatalytic activity is used as an indicator for selecting/ screening/ researching new cementitious materials having a predetermined level of photocatalytic activity.

## Patentansprüche

1. Verfahren zum Messen der photokatalytischen Aktivität eines zementartigen Materials, das Photokatalysatoren enthält, welches Verfahren umfasst, die elektromotorische Kraft zu messen, die zwischen zwei Elektroden erzeugt wird, die in Kontakt mit einem angestrahlten bzw. verdunkelten Bereich des Materials gebracht sind.

2. Verfahren nach Anspruch 1, wobei der Photokatalysator Titandioxid umfasst, das zumindest teilweise in Anatasform vorliegt.

3. Verfahren nach den Ansprüchen 1 bis 2, wobei der Photokatalysator vollvolumig im Material verteilt ist oder auf dessen Oberfläche schichtweise aufgebracht ist.

4. Verfahren nach den Ansprüchen 1 bis 3, wobei der Photokatalysator vollvolumig im Material oder in einer auf dessen Oberfläche schichtweise aufgebrachten Phase vorhanden ist, und zwar in einer Gewichtskonzentration zwischen 0,5% und 10%.

5. Verfahren nach den Ansprüchen 1 bis 4, wobei es sich bei dem zementartigen Material um Mörtel, Beton, Gips, eine zementartige Beschichtung oder eine zementartige Farbe handelt.

6. Verfahren nach den Ansprüchen 1 bis 5, wobei das zementartige Material ein Fertigungsgegenstand ist, z.B. ein Straßenpflaster oder ein Element hiervon, eine Wand oder ein Element hiervon, ein Tunnelgewölbe oder ein Element hiervon, eine Materialprobe, oder ein Musterstück ist, das von einem an Ort und Stelle eingesetzten Fertigungsgegenstand entnommen ist.

7. Verfahren nach den Ansprüchen 1, bis 6, wobei die elektromotorische Kraft mittels eines Multimeters gemessen wird, das an die beiden Elektroden angeschlossen ist.

8. Verfahren nach den Ansprüchen 1 bis 7, wobei der Photokatalysator Titandioxid ist und der angestrahlte Bereich mit einer Wellenlänge bestrahlt wird, die zwischen 350 nm und 400 nm liegt.

9. Verfahren nach den Ansprüchen 1 bis 8, kontinuierlich während einer vorbestimmten Zeitspanne ausgeführt.

10. Verfahren nach den Ansprüchen 1 bis 8, über eine Anzahl wiederholter Beobachtungen ausgeführt.

11. Verfahren nach den Ansprüchen 1 bis 10, wobei die elektromotorische Kraft einer von Photokatalysator freien Materialprobe, die zur geprüften Materialprobe äquivalent ist, aus der gemessenen elektromotorischen Kraft abgeleitet wird.

12. Verfahren nach den Ansprüchen 1 bis 11, wobei die gemessene Höhe der photokatalytischen Aktivität dazu verwendet wird, die Qualität frisch hergestellter zementartiger Materialien zu überwachen.

13. Verfahren nach den Ansprüchen 1 bis 11, wobei die gemessene Höhe photokatalytischer Aktivität dazu verwendet wird, die Qualität von zementartigen Materialien zu überwachen, die bereits an Ort und Stelle eingesetzt sind und Verschleiß erfahren haben.

14. Verfahren nach den Ansprüchen 1 bis 11, wobei die gemessene Höhe photokatalytischer Aktivität als Indikator zum Auswählen/Untersuchen/Entwickeln neuer zementartiger Materialien verwendet wird, die eine vorbestimmte Höhe photokatalytischer Aktivität haben.

## Revendications

1. Procédé destiné à mesurer l'activité photo catalytique d'un matériau cimentaire contenant des photocatalyseurs, qui comprend la mesure de la force électromotrice générée entre deux électrodes mises en contact respectivement avec une zone éclairée et une zone sombre dudit matériau.

2. Procédé selon la revendication 1, dans lequel le photocatalyseur comprend du dioxyde de titane, présent au moins en partie sous la forme d'anatase.

3. Procédé selon les revendications 1 à 2, dans lequel le photocatalyseur est dispersé en vrac dans le matériau ou est disposé en couches à sa surface.

4. Procédé selon les revendications 1 à 3, dans lequel le photocatalyseur est présent en vrac dans le matériau ou en phase disposée en couche à sa surface, dans une concentration en poids comprise entre 0,5 % et 10 %.

5. Procédé selon les revendications 1 à 4, dans lequel le matériau cimentaire est du mortier, du béton, du plâtre, un enduit cimentaire ou une peinture cimentaire.

6. Procédé selon les revendications 1 à 5, dans lequel le matériau cimentaire est un article de fabrication, par exemple un dallage ou un élément de celui-ci, un mur ou un élément de celui-ci, une voûte de tunnel ou un élément de celle-ci, un échantillon, ou un spécimen prélevé à partir d'un article de fabrication en place.

7. Procédé selon les revendications 1 à 6, dans lequel là force électromotrice est mesurée au moyen d'un multimètre connecté aux deux électrodes.

8. Procédé selon les revendications 1 à 7, dans lequel le photocatalyseur est du dioxyde de titane et la zone éclairée est éclairée avec une longueur d'onde comprise entre 350 nm et 400 nm.

9. Procédé selon les revendications 1 à 8, exécuté de manière continue tout au long d'une période de temps déterminée.

10. Procédé selon les revendications 1 à 8, exécuté par l'intermédiaire d'un certain nombre d'observations répétées.

11. Procédé selon les revendications 1 à 10, dans lequel la force électromotrice d'un échantillon sans photocatalyseur équivalent à celui qui est examiné est déduite de la force électromotrice mesurée.

12. Procédé selon les revendications 1 à 11, dans lequel le niveau de l'activité photocatalytique mesuré est utilisé pour surveiller la qualité des matériaux cimentaires nouvellement produits.

13. Procédé selon les revendications 1 à 11, dans lequel le niveau de l'activité photocatalytique mesuré est utilisé pour surveiller la qualité des matériaux cimentaires déjà en place et soumis à l'usure.

14. Procédé selon les revendications 1 à 11, dans lequel le niveau de l'activité photocatalytique mesuré est utilisé en tant qu'indicateur pour sélectionner / cribler / rechercher de nouveaux matériaux cimentaires présentant un niveau d'activité photo catalytique prédéterminé.
